Europäisches Patentamt

European Patent Office

Office européen des brevets

(11) Numéro de publication: **0 232 652**
**A1**

(12) # DEMANDE DE BREVET EUROPEEN

(21) Numéro de dépôt: **86402877.4**

(22) Date de dépôt: **19.12.86**

(51) Int. Cl.⁴: **A61K 37/18** , A61K 31/195

Le titre de l'invention a été modifié (Directives relatives à l'examen pratiqué à l'OEB, A-III, 7.3)

(30) Priorité: **19.12.85 FR 8518852**

(43) Date de publication de la demande:
**19.08.87 Bulletin 87/34**

(84) Etats contractants désignés:
**AT BE CH DE ES FR GB GR IT LI LU NL SE**

(71) Demandeur: **CENTRE NATIONAL DE LA RECHERCHE SCIENTIFIQUE (CNRS)**
**15, Quai Anatole France**
**F-75007 Paris(FR)**

(72) Inventeur: **Poupon, Marie-France**
**38, rue Emile Zola**
**F-94260 Fresnes(FR)**
Inventeur: **Echinard-Garin, Paule, Laure, Jeanne**
**31, rue Mozart**
**F-92330 Sceaux(FR)**
Inventeur: **Breillout, Fabienne, Rolande**
**5, rue Vercingétorix**
**F-75014 Paris(FR)**

(74) Mandataire: **Nony, Michel et al**
**Cabinet NONY & CIE 29, rue Cambacérès**
**F-75008 Paris(FR)**

(54) **Compositions nutritionnelles carencées en méthionine et supplémentées en homocystéine.**

(57) Composition nutritionnelle synthétique ou semi-synthétique destinée à inhiber le développement et la dissémination des tumeurs malignes chez les mammifères, caractérisée par le fait qu'elle est carencée en méthionine et supplémentée en homocystéine ; et utilisation, dans la préparation de telles compositions nutritionnelles, d'une source d'acides aminés carencée en méthionine.

EP 0 232 652 A1

**"Compositions nutritionnelles carencées en méthionine et supplémentée en homocystéine destinées à inhiber le développement et la dissémination des tumeurs malignes chez les mammifères".**

La présente invention a pour objet des compositions nutritionnelles destinées à inhiber le développement et la dissémination des tumeurs malignes, et en particulier des métastases chez les mammifères.

On sait que la dissémination métastatique des tumeurs malignes représente, dans l'évolution des cancers, une phase dont la prévention et le traitement s'avèrent très difficiles. La thérapeutique actuelle utilise la radiothérapie et la chimiothérapie, ainsi que la chirurgie lorsque les métastases sont peu nombreuses et accessibles. Toutefois, les résultats restent insuffisants dans de nombreux cas, et il apparaît donc nécessaire de rechercher de nouvelles therapeutiques.

On sait également que le métabolisme des cellules tumorales est différent de cellui des cellules normales et qu'en particulier la prolifération in vitro des cellules tumorales est dépendante de l'apport en méthionine exogène ; voir par exemple R.M. Hoffman, Biochem. Biophys. Acta 738, 49-87 (1984).

Bien que la méthionine soit considérée classiquement comme un acide aminé essentiel, la cellule normale, même en phase de prolifération, est peu sensible ou insensible à l'absence de méthionine lorsque celle-ci est remplacée notamment par l'homocystéine en présence de choline et de vitamine B12. En effet, les cellules normales peuvent dans ces conditions réaliser leur propre synthèse de méthionine qu'elles utilisent alors dans leur métabolisme. Les cellules tumorales en sont incapables.

On a maintenant découvert qu'une alimentation carencée en méthionine et supplémentée en homocystéine permet notamment d'inhiber dans des proportions significatives l'installation et le développement des métastases chez les mammifères.

La présente invention a pour objet une composition nutritionnelle synthétique ou semi-synthétique destinée à inhiber le développement et la dissémination des tumeurs malignes chez les mammifères, caractérisée par le fait qu'elle est carencée en méthionine, et supplémentée en homocystéine.

Parmi les compositions de l'invention, on citera en particulier celles qui correspondent à une carence en méthionine d'au moins 50%.

On ne peut pas définir dans l'absolu une composition carencée en méthionine, car les besoins en méthionine varient selon les espèces. Pour une espèce donnée, une composition carencée en méthionine peut être définie de la façon suivante. Soit une composition nutritionnelle équilibrée pour une espèce animale donnée, contenant y% en poids de méthionine. On considère qu'une composition est carencée à x% en méthionine lorsque ladite composition, qui est identique à la composition équilibrée pour tous les autres constituants, ne contient que

$$\frac{y(100 - x)}{100}\ \%$$

en poids de méthionine.

L'invention concerne en particulier une composition qui contient, comme source d'acides aminés, un mélange d'acides aminés essentiels en proportions convenables pour le mammifère considéré, mais avec une teneur réduite en méthionine, et dont les autres ingrédients sont exempts de protéines ou n'en apportent pas plus de 1% par rapport au poids sec total des constituants; parmi ces compositions, on citera en particulier celles dont la teneur en méthionine du mélange d'acides aminés utilisé est nulle.

Dans la composition de l'invention, l'homocystéine est ajoutée en remplacement de la méthionine, c'est-à-dire que la teneur en homocystéine correspond, en équivalents molaires, à la carence en méthionine.

La composition de l'invention, lorsqu'elle est destinée à être administrée par voie entérale, contient en outre des sources de glucides et de lipides, des sels minéraux, des vitamines et éventuellement des aliments de lest tels que des fibres végétales. La source d'acides aminés autres que l'homocystéine peut être dans ce cas constituée partiellement ou uniquement de protéines pauvres en méthionine, par exemple de protéines de soja ou d'hydrolysats de protéines de soja.

Une telle composition peut se présenter par exemple sous la forme de poudre, de suspension aqueuse ou d'émulsion, etc.....

Pour améliorer les propriétés organoleptiques des compositions destinées à la voie entérale, il est souhaitable que l'homocystéine soit présente, dans ces compositions (ou bien en tant que composition séparée à ajouter au moment de l'emploi) sous forme enrobée dans des microcapsules dont la paroi est soluble dans les voies digestives, par exemple sous l'action des enzymes digestives. Les matériaux constituant de telles microcapsules, ainsi que les procédés de préparation de telles microcapsules, sont connus en soi. On peut citer par exemple à cet égard le brevet français 2.527.438 qui décrit un procédé d'encapsulation à l'aide de microcapsules à parois constituées par des polyholosides réticulés.

La composition de l'invention, lorsqu'elle est destinée à être administrée par voie parentérale, se présente sous la forme d'une composition fluide homogène contenant, dans un véhicule aqueux isotonique, lesdits acides aminés, et éventuellement des glucides et des sels minéraux. De telles compositions peuvent être administrées, par exemple, chez l'homme, par voie intraveineuse. Les sels minéraux et les lipides peuvent être administrés séparément ou en même temps que les acides aminés, par voie intraveineuse. Les vitamines peuvent être administrées par exemple par voie intramusculaire.

Dans les régimes utilisant les compositions de l'invention, et en particulier dans le cas où les sources d'acides aminés sont des acides aminés synthétiques, il est nécessaire d'introduire de la choline. La choline peut être administrée séparément, par exemple avec les vitamines.

Bien entendu, les compositions de l'invention contiennent les substances nutritives et les véhicules ou adjuvants dans des quantités et des proportions relatives telles que les besoins quotidiens du mammifère traité soient assurés avec la prise d'un volume de composition acceptable pour le mammifère considéré. La proportion molaire d'homocystéine, correspond à celle de la méthionine manquante par rapport à une composition équilibrée pour l'espèce considérée.

Les besoins en glucides, lipides, acides aminés, sels minéraux et vitamines varient pour chaque espèce et sont soit connus soit déterminables selon les méthodes connues.

Les compositions de l'invention peuvent être administrées dès la découverte d'une tumeur, et en particulier avant ou immédiatement après un traitement chirurgical. Bien entendu, le traitement avec les compositions nutritionnelles carencées en méthionine sera généralement un traitement adjuvant associé aux autres moyens thérapeutiques tels que la chimiothérapie et la radiothérapie.

Le traitement peut être poursuivi par exemple pendant 1 à 4 mois. On peut poursuivre ensuite le traitement par un régime mixte de type végétarien.

L'invention a aussi pour objet un procédé de préparation des compositions telles que définies ci-dessus, par mélange des ingrédients en proportions convenables et éventuellement d'un véhicule approprié (eau ou sérum physiologique).

L'invention a également pour objet l'utilisation, dans la préparation de compositions nutritionnelles destinées à inhiber le développement et la dissémination des tumeurs malignes chez les mammifères, d'une source d'acides aminés carencée en méthionine, la méthionine manquante étant remplacée par l'homocystéine, comme indiqué ci-dessus.

L'invention concerne aussi un procédé de traitement thérapeutique, destiné à inhiber le développement et la dissémination des tumeurs malignes chez les mammifères, ce procédé consistant à alimenter le patient, par voie entérale ou parentérale, à l'aide d'une composition carencée en méthionine et supplémentée en homocystéine, telle que définie ci-dessus, administrée en quantité suffisante pour subvenir aux besoins nutritionnels.

Un des avantages de ce traitement est notamment qu'il permet de prévenir l'implantation et/ou de traiter les micrométastases ont on sait qu'elles sont peu sensibles à la chimiothérapie.

Les exemples suivants illustrent l'invention sans toutefois la limiter.


EXEMPLES 1 à 5 : Compositions semi-synthétiques

On a préparé une composition comprenant les ingrédients suivants (Tableau 1) :

TABLEAU 1

| Ingrédients nutritionnels | Exemple 1 Composition pour Souris ou Rat (g/kg de composition) |
|---|---|
| GLUCIDES : amidon de maïs prégélatinisé | 550 |
| PROTIDES : isolat de soja (carencé en méthionine) | 210 |
| LIPIDES : - Saindoux | 100 |
| - huile de noix | 10 |
| SELS MINERAUX COMPLETS | 50 |
| MELANGE VITAMINIQUE EQUILIBRE | 25 |
| FIBRES : agar-agar | 55 |

La composition de l'exemple 1 est carencée à 50% en méthionine.

Cette composition se présente sous la forme d'une poudre.

De façon analogue, on a préparé diverses compositions pour souris ou rats, chats, chiens ou hommes, assurant une carence en méthionine de 75% environ. Les teneurs de ces compositions (en g/kg de composition) sont résumées dans le Tableau 2 suivant.

4

TABLEAU 2

| Ingrédients nutritionnels | Exemple 2 Composition pour Souris ou Rat | Exemple 3 Composition pour Chat | Exemple 4 Composition pour Chien | Exemple 5 Composition pour l'Homme |
|---|---|---|---|---|
| GLUCIDES : amidon de maïs prégélatinisé | 620 | 615 | 685 | 675 |
| PROTIDES : isolat de soja | 120 | 175 | 125 | 120 |
| LIPIDES : - Saindoux | 100 | 100 | 100 | 110 |
| - huile de noix | 10 | 10 | 10 | 10 |
| SELS MINERAUX COMPLETS | 50 | 50 | 50 | 50 |
| MELANGE VITAMINIQUE EQUILIBRE | 25 | 25 | 25 | 25 |
| FIBRES : agar-agar | 75 | 25 | 5 | 10 |

Les sels minéraux complets utilisés dans les compositions précédentes sont indiqués dans le Tableau 3.

TABLEAU 3

| g/kg de composition | SOURIS RAT | CHAT | CHIEN | HOMME |
|---|---|---|---|---|
| Calcium | 2,78 | 16,4 | 22 | 3,35 |
| Phosphore | 8,25 | 11,49 | 13 | 2,75 |
| Sodium | 3,11 | 6 | 5,5 | 3,40 |
| Potassium | 6,41 | 7,4 | 6,3 | 4,05 |
| Chore | 2,76 | 7,3 | 2;6 | 0,46 |
| Magnésium | 1,29 | 1,89 | 2,5 | 0,87 |
| Soufre | 1,66 | 1,24 | 1,5 | 0,98 |
| **mg/kg de composition** | | | | |
| Manganèse | 1,2 | 121 | 56,5 | 8,4 |
| Zinc | 5,42 | 55 | 62,6 | 23 |
| Cuivre | 16 | 15 | 14,9 | 4,13 |
| Cobalt | 1 | 1 | 0,92 | 1 |
| Iode | 24 | 27 | 0,75 | 0,4 |
| Fer(ferreux) | 240 | 220 | 165 | 85 |
| Fluor | 0,6 | 0,45 | 0,5 | 0,4 |

Les mélanges vitaminiques équilibrés utilisés dans les compositions précédentes sont figurés dans le Tableau 4.

## TABLEAU 4

| mg/kg de composition | SOURIS RAT | CHAT | CHIEN | HOMME |
|---|---|---|---|---|
| A | 3,48 | 17,20 | 5,36 | 5,89 |
| D | 0,125 | 0,14 | 0,05 | 0,14 |
| B1 | 15,8 | 65 | 2,3 | 5,7 |
| B2 | 20 | 45 | 5 | 7,1 |
| B6 | 15,2 | 35 | 3 | 3,89 |
| B12 | 0,02 | 0,15 | 0,02 | 0,017 |
| E | 273 | 260 | 225 | 72 |
| K3 | 5 | 10 | 3 | 4 |
| PP | 25 | 44 | 48,6 | 63,4 |
| Acide pantothénique | 45,8 | 50 | 30 | 15 |
| Acide folique | 2 | 4 | 0,5 | 1,04 |
| biotine | 1 | 0,4 | 0,2 | 0,44 |
| Choline | 1500 | 3000 | 2000 | 1500 |
| C | 300 | 40 | 35 | 75 |
| INOSITOL | 850 | 300 | 300 | 236 |
| Acide paraamino-benzoïques | 100 | 100 | 100 | 100 |

EXEMPLE 6 : Composition synthétique pour la voie entérale.

De façon analogue on a préparé la composition suivante du tableau 5 (synthétique en ce qui concerne l'apport d'acides aminés). Les teneurs sont exprimées en g/kg de composition.

TABLEAU 5

| Ingrédients nutritionnels | Exemple 6 Composition pour Souris ou Rat |
|---|---|
| GLUCIDES : amidon de maïs prégélatinisé | 530 |
| PROTIDES : acides aminés de synthèse (sans méthionine) | 230 |
| LIPIDES : - Saindoux | 100 |
| - huile de noix | 10 |
| SELS MINERAUX COMPLETS | 50 |
| MELANGE VITAMINIQUE | 25 |
| FIBRES : agar-agar | 55 |

Les teneurs relatives en sels minéraux et en vitamines sont les mêmes que précédemment.
Les teneurs relatives en acides aminés sont indiquées dans le Tableau 6 suivant :

TABLEAU 6

| | | | |
|---|---|---|---|
| Arginine | 7,4 | Valine | 14,9 |
| Lysine | 15,9 | Histidine | 6,4 |
| Cystéine | 0,7 | Tyrosine | 12,4 |
| Tryptophane | 3,1 | Alanine | 6,6 |
| Glycocolle | 3,9 | Proline | 22,5 |
| (= glycine) | | Sérine | 11,0 |
| Isoleucine | 11,8 | Acide aspartique | 16,5 |
| Leucine | 20,2 | Acide glutamique | 51,7 |
| Phénylalanine | 11,4 | Homocystéine | 4,7 |
| Thréonine | 8,9 | | |

EXEMPLE 7 : Composition synthétique pour le traitement des humains par la voie parentérale, et son utilisation en association avec les éléments nutritionnels complémentaires.

Il s'agit d'une composition nutritionnelle classique d'acides aminés et de glucides pour la voie intraveineuse, mais la méthionine est remplacée par l'homocystéine.

La composition, sous forme d'une solution aqueuse, a la formule suivante (en g/l) :

GLUCIDES

-Sorbitol 40

PROTIDES

-L-Arginine* 6
-L-Lysine* 8
-L-Tryptophane 2,5
-Glycine 6
-L-Isoleucine 5,85
-L-Leucine 6,25
-L-Phénylalanine 9,60
-L-Thréonine 5
-L-Valine 7,20
-L-Histidine* 2
-L-Homocystéine 10

SELS MINERAUX

g/litre de composition Sodium (sous forme de chlorure) 0,350
* sous forme de chlorhydrate
Cette composition peut être administrée par voie intraveineuse.

Les éléments nutritionnels complémentaires peuvent être administrés comme décrit ci-après.

Les sels minéraux peuvent être administrés simultanément ou non, à l'aide des compositions classiques pour la voie intraveineuse, par exemple la composition IONITAN (marque déposée) ayant la formule suivante (g/l) :

SELS MINERAUX

-Sodium 1,495
-Potassium 1,872
-Calcium 0,16032
-Magnésium 0,04661
-Chlorure 2,722
- Acétate 0,590
-Sulfate 0,192
-Lactate 0,726
-Phosphate 0,245

De même, les lipides peuvent être administrés par voie intraveineuse à l'aide d'une composition classique telle que INTRALIPIDE (marque déposée) de formule suivante (g/l) :

LIPIDES

-huile de soja purifiée 100
-lécithine d'oeuf purifiée 12
-glycérol 22,5

Enfin les vitamines, y compris la choline, peuvent être administrées par voie intramusculaire. On peut utiliser par exemple une composition commerciale de vitamine B12 et une composition telle que l'OTONEURINE (marque déposée) ayant la formule suivante :

| OTONEURINE | g/1 |
|---|---|
| – Thiamine* ou vitamine $B_1$ | 2 |
| – Riboflavine Phosphate ou vitamine $B_2$ | 0,2 |
| – Pyridoxine * ou vitamine $B_6$ | 1 |
| – Nicotinamide ou vitamine PP | 8 |
| – Histidine * | 4 . |
| – Tryptophane | 2 . |
| – Citrate de Choline | 2 |

\* sous forme de chlorhydrate

## ETUDE PHARMACOLOGIQUE

On a étudié l'influence de la composition de l'exemple 1 sur l'apparition de métastases chez des souris $C_{57}$ B1/6.

On induit chez les souris la formation d'une tumeur primaire par greffage sous-cutané d'un fragment de tumeur 3LL ayant 1mm de diamètre.

En l'absence de traitement, les animaux meurent entre les jours 21 et 25. A l'autopsie, ils présentent des métastases pulmonaires multiples.

Dès la greffe tumorale, les souris ont été nourries exclusivement avec la composition de l'exemple 1, prise à volonté.

Les témoins étaient alimentés avec la composition suivante non carencée du tableau 7 suivant:

TABLEAU 7

| Ingrédients | % en poids |
|---|---|
| Glucides | 53 |
| Protides | |
| Caséine | 23 |
| Lait en poudre | 4 |
| Levure de Bière | 2,5 |
| Lipides | |
| Saindoux | 9,2 |
| Huile de noix | 0,8 |
| Sels minéraux | 5 |
| Vitamines | 2,5 |

Les traitements débutent dès la greffe et jusqu'à la fin de l'expérience.

Les résultats obtenus après 21 jours de traitement, sont résumés dans le Tableau 8 ci-après.

L'envahissement tumoral est déterminé par comptage des métastases à la surface des poumons. La note 100 est attribuée aux animaux dont les poumons sont complètement envahis par les métastases, rendant tout comptage impossible.

TABLEAU 8

|  | Nombre de métastases pulmonaires par animal | Médiane du nombre de métastases pulmonaires |
|---|---|---|
| Régime complet (traitement témoin) | 100-100-100-100-100 100-100-100-38-33-28 12-8-1 | 100 |
| Régime déprimé en méthionine | 100-100-62-52-41* 29-25-23-22-2-1-0 | 27 |

Test Wilcoxon : * p<0.01

On voit que la médiane du nombre de métastases a diminué de plus de 70% par rapport aux témoins.

Le traitement ne provoque ni pathologie carentielle, ni perte de poids, ni troubles de comportement.

On a obtenu des résultats analogues sur des rats WAG porteurs d'une greffe de sarcome J1 (sarcome du tissu pulmonaire). On a injecté à chaque rat, par voie sous-cutanée, $10^5$ cellules du clone J1. Dans ce cas, le traitement nutritionnel a commencé dès l'apparition de la tumeur, soit 15 jours après la greffe. En outre, les tumeurs ont été enlevées chirurgicalement lorsqu'elles atteignaient un diamètre de 14mm.


EXAMPLE 9 -BESOIN EN METHIONINE DE 2 CLONES D'UN RHABDOMYOSARCOME DE POTENTIEL METASTATIQUE DIFFERENT

Cellules utilisées :

-Lignée F9-4/0 de potentiel métastatique moyen : cette cellule F9-4/0 est une lignée établie à partie d'un rhabdomyosarcome induit chez le rat Wistar AG par injection intramusculaire de nickel.

Cette lignée à ensuite été clonée et a permis d'obtenir différents clones de potentiel métastatique varié (évalué par implantation par voie sous-cutanée chez le rat).

-Clone F9-4/21 de faible potentiel métastatique

-J9-4/1 très métastatique

-Fr : Fibroblastes embryonnaires de rat (cellules normales).

Milieu <u>de</u> <u>culture</u> :

Milieu Eagle modification Dulbecco sans méthionine (Eurobio).

Ce milieu est complémenté par 7.5 $\mu$M d'hydroxocobalamine, 0,1mM d'acide folique et 10% de sérum de veau foetal dialysé. L'homocystéine 0,1mM (milieu Met⁻Hcy +) est ajoutée à ce milieu de base.

<u>Protocole</u> :

Les cellules sont ensemencées dans des boîtes de Petri de 35mm à raison de 2.10⁴ cellules dans 2ml de milieu Met⁻Hcy⁺ +10% de sérum de veau foetal dialysé.

Différentes concentrations de méthionine sont alors ajoutées : 0-0,05 -0,1 -0,5 -0,75 -1 -1,5 -2 -2,5 -3 -4 -5 -et 30$\mu$g/ml.

Le nombre de cellules dans les boîtes de culture au bout de 4 jours est évalué à l'aide d'un compteur Coulter.

Les résultats sont exprimés en % de cellules présentes dans les boîtes à une concentration x de méthionine par rapport au nombre de cellules présentes dans l'échantillon témoin (30$\mu$m/ml).

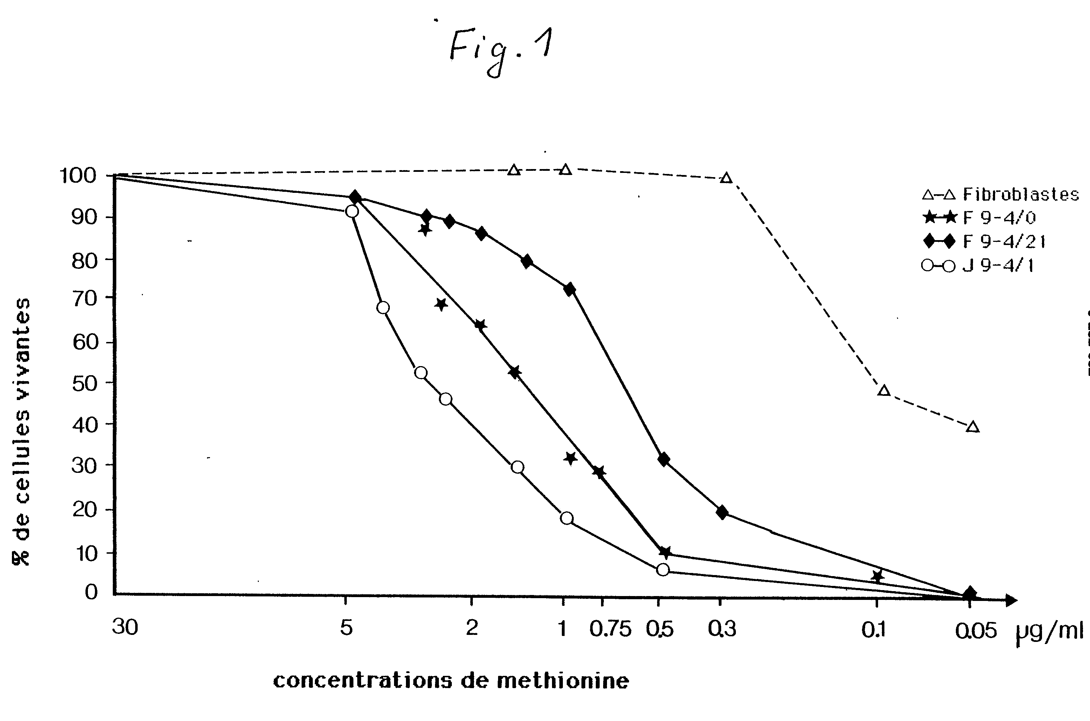

Ces résultats sont représentés sur la figure 1. On voit que les cellules fortement métastatiques sont plus sensibles à l'absence de méthionine, même en présence d'homocystéine.

Des résultats analogues ont été constatés avec les cellules fortement métastatiques suivantes (lignées humaines) :

MCF 7 adénocarcinome mammaire

HT 29 adénocarcinome colique

SCC 2 cancer du poumon à petites cellules

IGR 37 mélanome

A549 Asc mélanome

RAJI lymphome

## EXEMPLE 10 : ETUDE PHARMACOLOGIQUE CHEZ LE RAT

A) 2.10⁵ cellules J9-4/1 (fortement métastatiques) sont injectées en sous-cutanée. Lorsque les tumeurs atteignent 12mm de diamètre, les animaux sont traités par les différent régimes jusqu'à la fin de l'exprérience.

Les rats Wistar femelles (2 mois) ont été traités par des régimes dont la composition en protéines ou acides aminés est la suivante : Caséne 16%, Met⁻HCy + 16% ou Met⁻HCy-16%, lorsque la tumeur primaire atteignait un diamètre de 12mm et justqu'à la fin de l'expérience.

<u>Evolution</u> <u>de la</u> <u>tumeur</u> :

-la détection tumorale se fait environ 15 jours après la greffe ;

-la tumeur atteint 12·mm entre le 20ème et le 25ème jour et les métastases commencent à se transformer ;

-les ganglions axillaires linguinaux sont détectables au jour 30 ;

-les animaux meurent entre les jours 60 et 65.

A l'autopsie, ils présentent des métastases pulmonaires multiples.

Les résultats sont résumés :

1) dans le Tableau 9 :

Nombre de métastases pulmonaires présentes chez les animaux ;

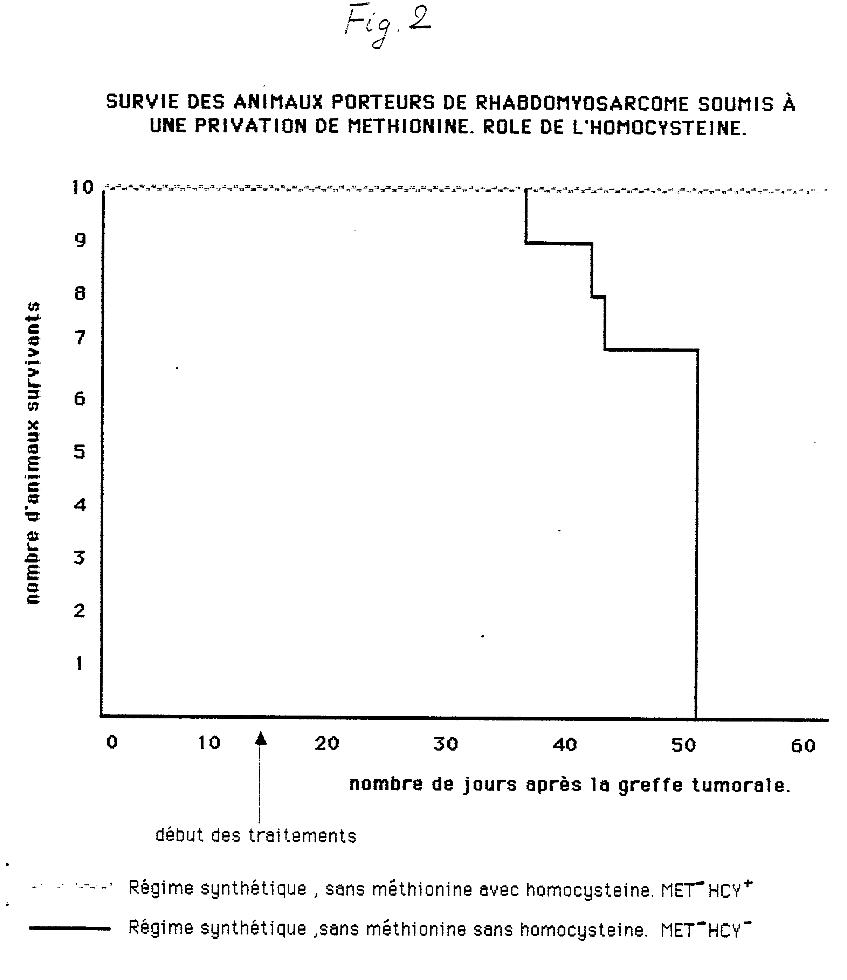

2) sur la figure 2 :

Courbe de survie des animaux soumis au régime Met⁻HCy⁻ comparée à celle des animaux traités par le régime Met⁻HCy⁺ ;

Met⁻HC⁺ : régime composé d'un mélange d'acides aminés de composition identique à la caséine. La méthionine est remplacée par l'homocystéine.

Met⁻HCy⁻ : régime composé d'un mélange d'acides aminés de composition identique à la caséine. Ce régime ne contient ni méthionine ni homocystéine.

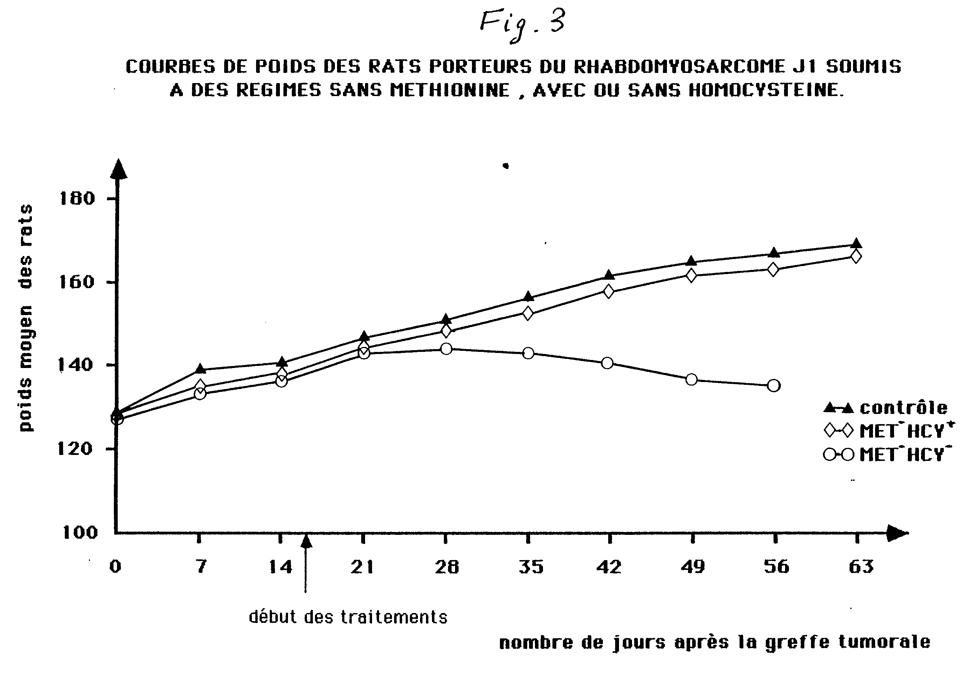

3) sur la figure 3 :
Courbe de poids des animaux soumis aux différents régimes.

CONCLUSIONS :

1) a) Le régime où la méthionine est substituée par l'homocystéine (Met⁻HCy⁺) entraîne une diminution significative du nombre de métastases pulmonaires.
b) Le régime totalement carencé en méthionine (Met⁻HCy⁻) entraîne une diminution très significative du nombre de métastases.
2) a) Le régime (Met⁻HCy⁺) est bien supporté par les animaux. Aucune perte de poids n'est détectable par rapport aux animaux témoins.
b) Le régime (Met⁻HCy⁻) provoque une chute brutale du poids des animaux 10 jours après le début du traitement.
3) Les animaux soumis au régime Met⁻HCy⁻ meurent plus rapidement que les rats soumis au régime Met⁻HCy⁺.
4) Cette expérience, qui intervient à un stade précoce, correspond en fait au traitement ou à la prévention des micrométastases.

TABLEAU 9

| | Nombre de métastases pulmonaires par animal | Médiane du nombre de métastases pulmonaires |
|---|---|---|
| Controle Caseine 16% | 2-12-14-17-20-26-27-28-28-38 40-41-44-46-50-72-88-97-111-115 | 39 (2-115) |
| MET- HCY+ 16% | 1-6-10-12-12-12-12-14-14-15-18-22-25-32-36-41-51-57-65-83 * $p < 0.05$ | 17 (1-83) |
| MET- HCY- 16% | 0-0-0-0-3-3-4-7-12 * $p < 0.01$ | 3 (0-12) |

B) Dans une autre expérience on a obtenu les résultats suivants :

14

## TABLEAU 10

| | nombre de métastases pulmonaires par rat | médiane du nombre de métastases |
|---|---|---|
| Caséine 16% | 9-11-14-17-26-26 35-50-72-73 | 26 |
| MET- HCY+ 16% | 1-6-6-6-9-13-13 32-32 | 9 |
| | $p < 0.02$ | |

Dans les expériences précédentes, le mélange nutritionnel avait la composition suivante :

TABLEAU 11

COMPOSITION DE BASE

| Ingrédients nutritionnels | g/Kg de composition |
|---|---|
| GLUCIDES : amidon de maïs prégélatiné | 685 |
| PROTIDES : caséine | 160 |
| ou mélange d'acides aminés | 160 |
| LIPIDES : huile de maïs | 10 |
| SELS MINERAUX : | 55 |
| MELANGE VITAMINIQUE : comprenant la choline l'acide folique et la vitamine B 12 | 27 |
| FIBRES : agar-agar | 50 |
| EXCIPIENT : monostéarate de glycérol | 13 |

C) Dans une autre expérience, avec un régime analogue mais dans lequel on a remplacé les mélanges d'acides aminés soit par la caséine, soit par des protéines de soja (pauvre en méthionine) on a obtenu les résultats suivants (Tableau 12) :

TABLEAU 12

|  | Nombre de métastases pulmonaires par animal | Médiane du nombre de métastases pulmonaires |
|---|---|---|
| Caséine 10% a | 8-24-26-33-35-42 45-54-75 ns | 39 |
| Soja 12% a | 2-13-17-18-26-28[c] 28-33-73    p<0,01 | 27 |
| Soja 12% b + méthionine | 27-30-32-36-38-[c] 43-44-59 ns | 38 |

a) le régime 10% caséine est équivalent au régime 12% soja d'un point de vue nutritionnel.

b) la quantité de méthionine dans ce régime est de 0,4% de composition nutritionnelle.

c) la différence entre ces deux groupes est statistiquement significative p<0.02 test de Wilcoxon.

CONCLUSIONS :

. L'addition de méthionine dans le régime soja 12% annule l'effet de ce régime sur la dissémination métastatique du rhabdomyosarcome.

. La diminution de méthionine dans le régime de rats porteurs de tumeur semble être responsable de cet effet.

EXEMPLE 11 : COMPOSITION SYNTHETIQUE D'ACIDES AMINES

Composition de l'apport protéique (homme)

17

| Acides aminés essentiels | mg/g de composition |
|---|---|
| L Isoleucine | 40 |
| L Leucine | 70 |
| L Lysine | 55 |
| L Cystéine | 5 |
| L Phénylalanine | 40 |
| L Tyrosine | 30 |
| DL Thréonine | 40 |
| L Tryptophane | 10 |
| L Valine | 50 |
| DL Homocystéine | 20 |
| Acides aminés non essentiels | |
| L Arginine | 80 |
| L Histidine | 30 |
| L Alanine | 60 |
| L Ac. Aspartique | 110 |
| L Ac. Glutamique | 190 |
| Glycine | 20 |
| L Proline | 100 |
| L Sérine | 50 |

Cette composition, qui peut être diluée dans l'eau, est administrée à raison de 1g/kg de poids/jour, à titre d'apport protéique.

**Revendications**

1. Composition nutritionnelle synthétique ou semi-synthétique destinée à inhiber le développement et la dissémination des tumeurs malignes chez les mammifères, caractérisée par le fait qu'elle est carencée en méthionine et supplémentée en homocystéine.

2. Composition selon la revendication 1, caractérisée par le fait qu'elle est carencée en méthionine dans une proportion d'au moins 50%.

3. Composition selon l'une quelconque des revendications précédentes, caractérisé par le fait qu'elle contient, comme source d'acides aminés, un mélange d'acides aminés essentiels en proportions convenables pour le mammifère considéré, mais avec une teneur réduite en méthionine, et que les autres ingrédients sont exempts de protéines ou n'en apportent pas plus de 1% par rapport au poids total sec des constituants.

4. Composition selon la revendication 3, caractérisée par le fait que la teneur en méthionine dudit mélange d'acides aminés est nulle.

5. Composition selon l'une quelconque des revendications précédentes, caractérisée par le fait qu'elle contient de l'homocystéine en proportion molaire correspondant à la méthionine manquante.

18

6. Composition selon l'une quelconque des revendications 3 à 5, caractérisée par le fait qu'elle contient en outre des sources de glucides et de lipides, des sels minéraux, des vitamines, et éventuellement des fibres végétales, et qu'elle constitue une composition pour la voie entérale.

7. Composition selon la revendiation 6, caractérisée par le fait que l'homocystéine est enrobée dans des micro-capsules dont la paroi est soluble dans les voies digestives.

8. Composition selon l'une quelconque des revendications 3 à 5, caractérisée par le fait qu'elle contient, dans un véhicule aqueux isotonique, lesdits acides aminés, et éventuellement des glucides et des sels minéraux, et qu'elle constitue une composition pour la voie parentérale.

9. Composition selon l'une quelconque des revendications 1 et 2, caractérisée par le fait qu'elle contient, comme source partielle ou unique d'acides aminés, des protéines pauvres en méthionine, et qu'elle constitue une composition pour la voie entérale.

10. Composition selon la revendication 9, caractérisée par le fait que l'homocystéine est présente, soit séparément, soit en mélange avec les autres ingrédients, sous forme enrobée dans des micro-capsules dont la paroi est soluble dans les voies digestives.

11. Utilisation, dans la préparation de compositions nutritionnelles destinées à inhiber le développement et la dissémination des tumeurs malignes chez les mammifères, d'une source d'acides aminés carencée en méthionine et supplémentée en homocystéine.

12. Utilisation selon la revendication 11, caractérisée par le fait que la source d'acides aminés autres que l'homocystéine est constituée partiellement ou totalement de protéines pauvres en méthionine.

13. Procédé de traitement thérapeutique destiné à inhiber le développement et la dissémination des tumeurs malignes chez les mammifères, consistant à alimenter le patient, par voie entérale ou parentérale, à laide d'une composition carencée en méthionine et supplémentée en homocystéine, en quantité suffisante pour subvenir aux besoins nutritionnels.

Fig. 1

△—△ Fibroblastes
★—★ F 9-4/0
◆—◆ F 9-4/21
○—○ J 9-4/1

% de cellules vivantes

concentrations de methionine

0 232 652

0 232 652

Fig. 2

SURVIE DES ANIMAUX PORTEURS DE RHABDOMYOSARCOME SOUMIS À UNE PRIVATION DE METHIONINE. ROLE DE L'HOMOCYSTEINE.

nombre d'animaux survivants

nombre de jours après la greffe tumorale.

début des traitements

- - - - - - Régime synthétique , sans méthionine avec homocysteine. MET⁻HCY⁺

———— Régime synthétique ,sans méthionine sans homocysteine. MET⁻HCY⁻

# Fig. 3

**COURBES DE POIDS DES RATS PORTEURS DU RHABDOMYOSARCOME J1 SOUMIS
A DES REGIMES SANS METHIONINE , AVEC OU SANS HOMOCYSTEINE.**

Office européen
des brevets

RAPPORT PARTIEL
DE RECHERCHE EUROPEENNE
qui selon la règle 45 de la Convention sur le brevet
européen est considéré, aux fins de la procédure ultérieure,
comme le rapport de recherche européenne

Numéro de la demande

EP 86 40 2877

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | FR - A - 2 434 622 (OTSUKA PHARMACEUTICAL FACTORY, INC.) | | A 61 K 37/18 A 61 K 31/195 |
| | * Revendications 1-6; page 4, lignes 28-38; page 5, lignes 1-13; exemples * | 1-4,11 | |
| A | | 6,8,9, 12 | |
| | -- | | |
| Y | DE - A - 3 329 253 (MILUPA AG) | | |
| | Revendications 1-4 * | 1-4,11 | |
| | -- | | |
| Y | BRITISH JOURNAL OF CANCER, vol. 42, no. 1, 1980, pages 121-128, GB; M.J. TISDALE: "Effect of methionine deprivation on methylation and synthesis of macromolecules" | | |
| | * Page 121, résumé et colonne de gauche, lignes 1-8; page 123, colonne de gauche, moitié 2 - colonne de droite, moitié 1; page 126, colonne de droite, dernier alinéa - page 127, | 1,5,11 | |

DOMAINES TECHNIQUES RECHERCHES (Int. Cl.4)

A 61 K
A 23 L
A 23 K

### RECHERCHE INCOMPLETE ./.

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes 1-12

Revendications ayant fait l'objet de recherches incomplètes.

Revendications n'ayant pas fait l'objet de recherches 13

Raison pour la limitation de la recherche:

Méthode de traitement chirurgical ou thérapeutique du corps humain ou animal (voir art. 52(4) de la convention sur le brevet européen).

| Lieu de la recherche | Date d'achèvement de la recherche | Examinateur |
|---|---|---|
| La Haye | 17-03-1987 | DEKEIREL |

OEB Form 1505.1 03.82

| DOCUMENTS CONSIDERES COMME PERTINENTS | | | CLASSEMENT DE LA DEMANDE (Int. Cl. 4) |
|---|---|---|---|
| Catégorie | Citation du document avec indication, en cas de besoin, dés parties pertinentes | Revendica-tion concernée | |
| Y | colonne de gauche, ligne 11 *<br>--<br>FR - A - 2 244 468 (PASSWATER)<br><br>* Revendications 1,8-10; page 6, ligne 22 *<br>-- | 1,5,11 | |
| A | CHEMICAL ABSTRACTS, vol. 103, no. 3, 9 décembre 1985, page 35, abrégé 189 318u, Columbus, Ohio, US K.S. McCULLY et al.: "Antineo-plastic activity of N-maleamide homocysteine thiolactone amide encapsulated within liposomes" & PROC.SOC.EXP.BIOL.MED.1985, 180(1), 57-61<br><br>* Résumé * -- | | |
| A | PATENT ABSTRACTS OF JAPAN, vol. 7, no. 14, (C-146)(1159), 20 janvier 1983, Tokio, JP; & JP - A - 57 171 920 (YOSHIO MURAI) 22-10-1982<br><br>* Abrégé * | 1,3,7, 11<br><br>1 | DOMAINES TECHNIQUES RECHERCHES (Int. Cl.4) |
| A | ANNUAL REVIEW OF BIOCHEMISTRY, vol. 52, 1983, pages 187-222, US; A.J.L. COOPER: "Biochemistry of sulfur-containing amino acids"<br><br>* Page 215, lignes 8-19 *<br>-- | 1,5,11 | |
| D,A | BIOCHIMICA ET BIOPHYSICA ACTA, No. 738, 1984, pages 49-87 Elsevier Science Publishers B.V. NL; R.M. Hoffman: "Altered methionine metabolism, DNA methylation and oncogene expres-sion in carcinogenesis. A review and synthesis."<br>-- | | |
| D,A | FR - A - 2 527 438 (CENTRE NATIONAL DE LA RECHERCHE SCIENTI-FIQUE (CNRS)<br><br>------------------- | | |